# EUROPEAN PATENT SPECIFICATION

(11) **EP 2 934 379 B1**
(45) Date of publication and mention of the grant of the patent: **08.02.2017**
(21) Application number: 13818131.8
(22) Date of filing: 18.12.2013
(51) Int. Cl.: A61F 2/08, A61B 17/04, A61B 17/06

(54) **APPARATUS FOR STITCHING TENDONS**
VORRICHTUNG ZUM NÄHEN VON SEHNEN
APPAREIL DE SUTURE DE TENDONS

(30) Priority: 19.12.2012 US 201213720648
(43) Date of publication of application: 28.10.2015
(73) Proprietor: Biomet Sports Medicine, LLC, Warsaw, Indiana 46582 (US)
(72) Inventor: DENHAM, Gregory, J., Warsaw, IN 46582 (US); BERELSMAN, Brian, K., Warsaw, IN 46580 (US)
(74) Representative: Mays, Julie
(86) International application number: PCT/US2013/075989
(87) International publication number: WO 2014/100109

(56) References cited:
- US-A1- 2011 098 727
- US-A1- 2012 046 693
- US-A1- 2012 059 417

## Description

### FIELD

The present disclosure relates to methods and apparatuses for stitching tendons.

### BACKGROUND

This section provides background information related to the present disclosure which is not necessarily prior art.

Tears caused by trauma or disease in soft tissue, such as cartilage, ligament, or muscle, can be repaired by suturing. A suture construct may be used to secure the soft tissue to bone. One end of the suture construct may be secured to the soft tissue using stitches, and the other end of the suture construct may be secured to the bone using an anchor. The suture construct may include an adjustable loop including strands that may be pulled to reduce the size of the adjustable loop and thereby bring the soft tissue closer to bone.

Occasionally, the stitches securing the suture construct to the soft tissue may pull through the tissue. In particular, the stitch closest to the end of the tissue tear, adjacent to the anchor, may pull through the tissue, resulting in considerable movement of the soft tissue with respect to its original location in or on the bone. Thus, there is a need in the relevant art for tissue repair techniques and associated devices for facilitating suturing and stitching while preventing stitches from pulling through tissue.

### SUMMARY

This section provides a general summary of the disclosure, and is not a comprehensive disclosure of its full scope or all of its features.

Methods and apparatuses for repairing a tear in soft tissue are disclosed. A method according to the principles of the present disclosure includes connecting an intermediate member to a bone anchor and placing the intermediate member on the soft tissue. The method further includes inserting a first suture through the intermediate member and the soft tissue to attach the intermediate member to the soft tissue and fixing the bone anchor to bone to secure the soft tissue to the bone.

The invention is disclosed in the appended claims.

Further areas of applicability will become apparent from the description provided herein. The description and specific examples in this summary are intended for purposes of illustration only and are not intended to limit the scope of the present disclosure.

### DRAWINGS

The drawings described herein are for illustrative purposes only of selected embodiments and not all possible implementations, and are not intended to limit the scope of the present disclosure.
FIG. 1 is a perspective view of a first example of an intermediate member routed through a flexible anchor and placed on one side of soft tissue, with stiches extending through the intermediate member and into the soft tissue;
FIG. 2 is a side view of the intermediate member and soft tissue connected to the flexible anchor using a suture construct including an adjustable loop;
FIG. 3 is a top view of the intermediate member, soft tissue, flexible anchor, and suture construct of FIG. 2;
FIG. 4 is a side view of the intermediate member, soft tissue, flexible anchor, and suture construct of FIG. 2, with the intermediate member placed around the soft tissue and secured to the soft tissue using stiches;
FIG. 5 is a side view similar to that shown in FIG. 4 but with the intermediate member secured to the soft tissue using flexible anchors arranged in an inline configuration;
FIG. 6 is a side view similar to that shown in FIG. 4 but with the intermediate member secured to the soft tissue using flexible anchors arranged in a u-shaped configuration;
FIGS. 7A and 7B are perspective views of adjustable loops extending through soft tissue and a second example of an intermediate member, with locking members inserted through the adjustable loops;
FIGS. 8 and 9 are perspective views of a third example of an intermediate member connected to a flexible anchor using an adjustable loop, the intermediate member including needles for additional fixation to soft tissue;
FIG. 10 is a perspective view of a fourth example of an intermediate member connected to a flexible anchor using an adjustable loop, with stiches extending through the intermediate member and into soft tissue;
FIGS. 11 and 12 are perspective views of a fifth example of an intermediate member including an adjustable loop that is routed through a flexible anchor to secure the soft tissue to the flexible anchor; and
FIGS. 13A and 13B are side views of the intermediate member, soft tissue, flexible anchor, and suture construct of FIGS. 11 and 12, with the flexible anchor inserted into bone to secure the soft tissue to the bone.

Corresponding reference numerals indicate corresponding parts throughout the several views of the drawings.

### DETAILED DESCRIPTION

Example embodiments will now be described more fully with reference to the accompanying drawings.

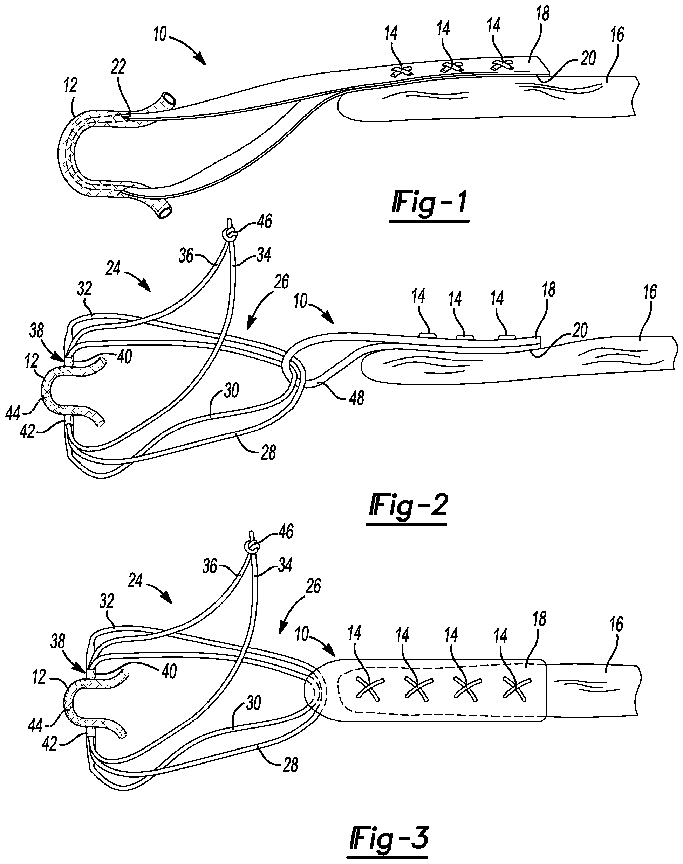

Referring to FIG. 1, an intermediate or load-dispersing member 10 is shown routed through a flexible anchor 12, with stiches 14 extending through the intermediate member 10 and into soft tissue 16 to secure the intermediate member 10 to the soft tissue 16. The arrangement shown in FIG. 1 may be used to repair a tear in the soft tissue 16 such as by attaching the soft tissue 16 to another piece of soft tissue or to bone. Instead of applying tension directly to the stitches 14 when attaching the soft tissue 16 to another piece of soft tissue or to bone, tension may be applied to the intermediate member 10, which in turn applies tension to the stiches 14. The intermediate member 10 aids in the distribution of load on the stitches 14 to increase the stiffness and maximum failure strength of the repair. In this regard, the intermediate member 10 is configured to increase pull-out forces of the stitches 14 and transfer load from those of the stitches 14 disposed closest to the flexible anchor 12 to those of the stitches 14 disposed further away from the flexible anchor 12. The intermediate member 10 may be folded over to form first and second segments 18, 20, both of which may be placed on one side of the soft tissue 16.

The intermediate member 10 may be a flat, planar ribbon or sheath having a width that is greater than its thickness. The intermediate member 10 may be sufficiently strong to prevent the stitches 14 from being pulled therethrough without tearing the intermediate member 10 but flexible enough to enable manipulation thereof. The intermediate member 10 may be made from polyester, polyethylene, polyurethane urea, and/or bioresorbable material. The intermediate member 10 may be braided, knit, or woven and/or may include a mesh or matrix such as a SportMesh™ Soft Tissue Reinforcement, available from Arthrotek®, a Biomet® company of Warsaw, Indiana.

The flexible anchor 12 defines a passage 22 through which the intermediate member 10 is routed. The flexible anchor 12 may be inserted into a hole drilled into bone, and tension may be applied to first and second segments 18, 20 of the intermediate member to set the flexible anchor 12. In turn, the flexible anchor 12 engages the portion of the bone surrounding the hole and thereby secures the soft tissue 16 to the bone. The flexible anchor 12 may be a JuggerKnot™ Soft Anchor, available from Biomet® of Warsaw, Indiana. Further examples of flexible anchors are disclosed in U.S. Patent Application Publication No. 2011/0264141, filed on July 6, 2011, which is incorporated by reference herein in its entirety.

Referring to FIGS. 2 and 3, the intermediate member 10 is shown connected to the flexible anchor 12 using a preformed adjustable self-locking suture construct 24 that includes a tensioning member 26 such as a double-loop configuration having two adjustable loops 28, 30. The tensioning member 26 may incorporate Ziploop™ Technology, available from Biomet of Warsaw, Indiana. The tensioning member 26 includes a suture 32 having a first end 34 and a second end 36. The suture 32 includes a braided body 38 having a first end 40 and a second end 42 and defining a passage 44 therein.

To form the suture construct 24, the first end 34 of the suture 32 can be inserted through the passage 22 in the flexible anchor 12 to position the first and second ends 40, 42 of the braided body 38 on opposite sides of the passage 22 as shown. The first end 34 of the suture 32 can then be inserted through the first end 40 of the braided body 38, through the passage 44 in the braided body 38, and out the second end 42 of the braided body 38 to form the adjustable loop 28. The second end 36 of the suture 32 can then be inserted through the second end 42 of the braided body 38, through the passage 44 in the braided body 38, and out the first end 40 of the braided body 38 to form the adjustable loop 30. The first and second ends 34, 36 can then be tied together to form a knot 46 as shown or left as separate strands. The flexible anchor 12 and the suture construct 24 can be provided together with the suture construct 24 formed as shown.

To secure the soft tissue 16 to bone, the intermediate member 10 can be inserted partially through the adjustable loops 28, 30, folded, and placed on one side of the soft tissue 16 to form a loop 48. The stitches 14 can then be formed through the intermediate member 10 and the soft tissue 16 to fix the intermediate member 10 to the soft tissue 16. The flexible anchor 12 can then be fixed within the bone, and the first and second ends 34, 36 of the suture 32 can be pulled to decrease the size of the adjustable loops 28, 30. As the size of the adjustable loops 28, 30 decreases, the soft tissue 16 is pulled closer to the location where the flexible anchor 12 is fixed to the bone.

In various implementations, the suture construct 24 may include multiple adjustable loops and/or multiple braided bodies arranged in various configurations. Further examples of suture constructs are disclosed in U.S. Patent No. 7,601,165, filed on September 29, 2006, and U.S. Patent No. 7,959,650, filed on August 22, 2008. Further examples of suture constructs incorporating flexible anchors are disclosed in U.S. Patent No. 7,905,904, filed on January 15, 2008, and U.S. Patent Application Publication No. 2011/0098727 ("the '727 publication"), filed on October 29, 2010. An exemplary embodiment of a preformed adjustable self-locking suture construct including a double-loop configuration is disclosed in FIG. 14 of the '727 publication, although other embodiments of the '727 publication may be employed.

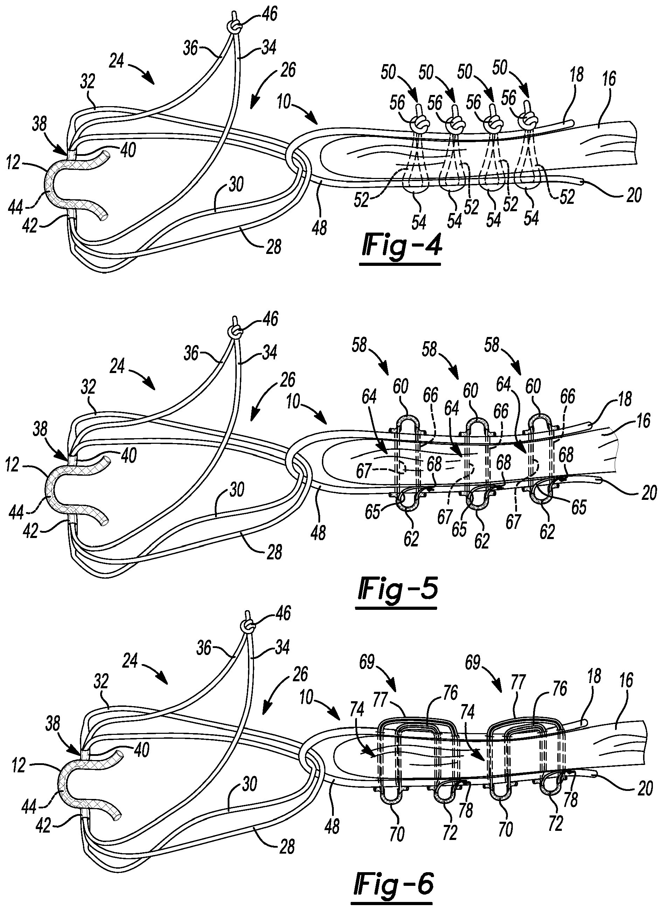

Referring to FIG. 4, the first and second segments 18, 20 of the intermediate member 10 are shown placed on opposite sides of the soft tissue 16 and secured to the soft tissue 16 using stiches 50. To form each of the stiches 50, a suture 52 may be passed through the first segment 18, the soft tissue 16, and the second segment 20 in one direction. The suture 52 may then be passed back through the first segment 18, the soft tissue 16, and the second segment 20 in the opposite direction to form a loop 54. Opposite ends of the suture 52 may then be tied together to form a knot 56.

Referring to FIG. 5, the intermediate member 10 is shown wrapped around the soft tissue 16 with the first and second segments 18, 20 placed on opposite sides of the soft tissue 16 and secured to the soft tissue 16 using preformed adjustable self-locking suture constructs 58 arranged in an inline configuration. Each of the suture constructs 58 includes flexible anchors 60, 62 positioned on opposite sides of the soft tissue 16 and held together using a tensioning member 64. Each of the tensioning members 64 can include a suture 65 arranged in a double-loop configuration with two adjustable loops 66, 67 and ends 68. The tensioning members 64 may incorporate Ziploop™ Technology, available from Biomet of Warsaw, Indiana.

Each of the suture constructs 58 may be preformed by passing the ends 68 of the suture 65 through passages in the flexible anchors 60, 62 in a manner similar to that described above with respect to the suture construct 24. One of the flexible anchors 60, 62 may then be pushed through the soft tissue 16 to position the flexible anchors 60, 62 on opposite sides of the soft tissue 16. The ends 68 of the suture 65 may then be pulled to decrease the size of the adjustable loops 66, 67 and thereby bring the first and second segments 18, 20 of the intermediate member 10 closer to each other. The ends 68 of the suture 65 may then be tied together as shown or left as separate strands. An exemplary suture construct including a double-loop configuration passed through two flexible anchors is disclosed in FIG. 14 of the '727 publication.

Referring to FIG. 6 the intermediate member 10 is shown wrapped around the soft tissue 16 with the first and second segments 18, 20 placed on opposite sides of the soft tissue 16 and secured to the soft tissue 16 using preformed adjustable self-locking suture constructs 69 arranged in a u-shaped configuration. Each of the suture constructs 69 includes flexible anchors 70, 72 positioned on the same side of the soft tissue 16 and held together using a tensioning member 74. Each of the tensioning members 74 includes a suture 75 arranged in a double-loop configuration with adjustable loops 76, 77 and ends 78. The tensioning members 74 may incorporate Ziploop™ Technology, available from Biomet of Warsaw, Indiana.

Each of the suture constructs 69 may be preformed by passing the ends 78 of the suture 75 through passages in the flexible anchors 70, 72 in a manner similar to that described above with respect to the suture constructs 58. One of the flexible anchors 70, 72 may then be pushed through the soft tissue 16 in one direction and then pushed through the soft tissue 16 again in the opposite direction to position the flexible anchors 70, 72 on the same side of the soft tissue 16. The ends 78 of the suture 75 may then be pulled to decrease the size of the adjustable loops 76, 77 and thereby bring the first and second segments 18, 20 of the intermediate member 10 closer to each other. The ends 78 of the suture 75 may then be tied together as shown or left as separate strands.

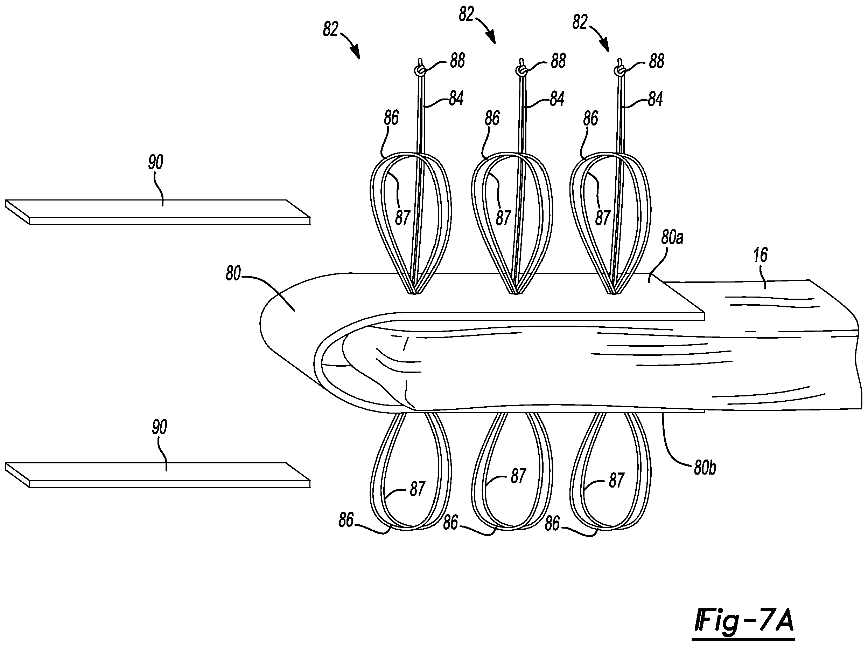

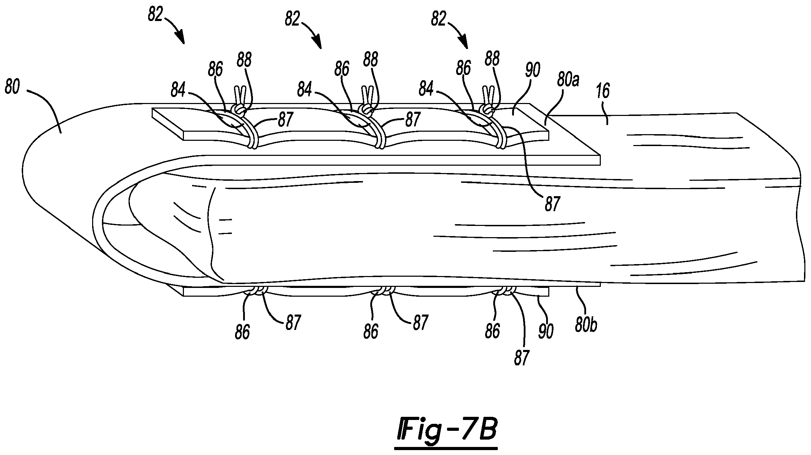

Referring to FIGS. 7A and 7B, an intermediate member 80 is shown wrapped around the soft tissue 16 to form spaced apart segments 80a, 80b with the soft tissue 16 disposed between the segments 80a, 80b and suture constructs 82 extending through the segments 80a, 80b and the soft tissue 16. Each of the suture constructs 82 includes a suture 84 arranged in a double-loop configuration with two adjustable loops 86, 87 and ends 88. The suture constructs 82 may incorporate Ziploop™ Technology, available from Biomet of Warsaw, Indiana. An exemplary suture construct including two adjustable loops is disclosed in FIG. 4A of U.S. patent number 7,601,165.

Each of the suture constructs 82 may be preformed in a manner similar to that described above with respect to the suture construct 24. The adjustable loops 86, 87 may then be pushed partially through the intermediate member 80 and the soft tissue 16 to position portions of the adjustable loops 86, 87 on both opposite sides of the soft tissue 16 as shown. The ends 88 of the suture 84 may then be pulled to decrease the size of the adjustable loops 86, 87. The ends 88 may then be tied together as shown or left as separate strands.

Locking members 90 may be inserted into the adjustable loops 86 to prevent the adjustable loops 86 from being pulled through the intermediate member 80 when the ends 88 are pulled. The locking members 90 may have sufficient rigidity and size to prevent the adjustable loops 86 from being pulled through the intermediate member 80 but flexible enough to enable manipulation thereof. The locking members 90 may be strips, rods, or sutures, may be made from polyester, polyethylene, and/or polyether ether ketone (PEEK), and may have a thickness or diameter between 0.25 millimeters (mm) and 0.5 mm. Further examples of suture constructs and locking members are disclosed in U.S. Patent Application Publication No. 2011/0208240 (see, e.g., FIGS. 4 through 6), filed on May 2, 2011.

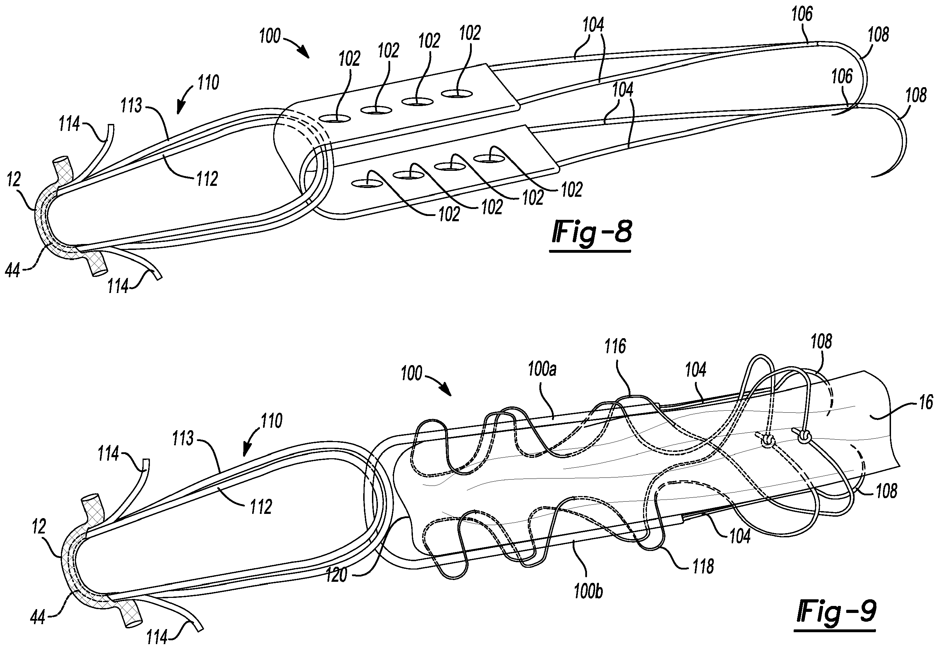

Referring to FIG. 8, an intermediate member 100 defines a plurality of bifurcations or openings 102. The intermediate member 100 may be a flat, planar ribbon or sheath having a width that is greater than its thickness. The intermediate member 100 may be formed (e.g., braided) from polyester, polyethylene, polyurethane urea, and/or bioresorbable material. Each end of the intermediate member 100 has suture ends 104 extending therefrom and converging at a connection 106 between the suture ends 104 and a needle 108. The suture ends 104 may be incorporated as part of the intermediate member 100 or be separate and passed through passages within the intermediate member 100. In one example, the intermediate member 100 can be formed by braiding portions of a plurality of strands and the unbraided portions of the strands may form the suture ends 104.

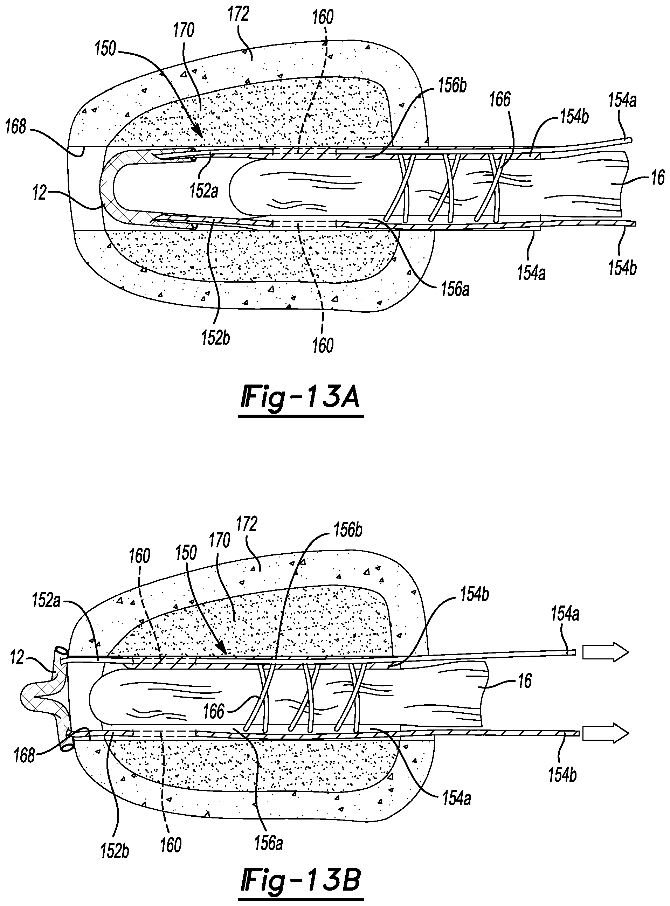

The intermediate member 100 is shown connected to the flexible anchor 12 using a preformed adjustable self-locking suture construct 110 that includes a double-loop configuration with two adjustable loops 112, 113 and ends 114. The suture construct 110 may be formed in the manner described above with respect to the suture construct 24. When the flexible anchor 12 is secured to bone, the ends 114 may be pulled to decrease the size of the adjustable loop 112 and thereby bring the intermediate member 100 closer to the flexible anchor 12. Further examples of suture constructs including adjustable loops and needles are disclosed in U.S. Patent Application Publication No. 2012/0046693 (see, e.g., FIG. 13), filed on November 3, 2011.

Referring to FIG. 9, the intermediate member 100 may be placed around the soft tissue 16 to form spaced-apart segments 100a, 100b with the soft tissue 16 disposed therebetween, and the needles 108 may be inserted into the soft tissue 16 to secure the intermediate member 100 to the soft tissue 16. Sutures 116, 118 may then be passed through the openings 102 as the sutures 116, 118 are stitched in and out of the soft tissue 16. The sutures 116, 118 are stitched in and out of the soft tissue 16 towards a distal end 120 of the soft tissue 16 on one side of the soft tissue 16 and then stitched back in the opposite proximal direction on the opposite side of the soft tissue 16. The ends of the suture 116 may then be tied to the ends of the suture 118 as shown and the needles 108 may be cut.

Instead of placing the intermediate member 100 around the soft tissue 16 as shown, both ends of the intermediate member 100 may be placed on one side of the soft tissue 16 in a manner similar to that shown in FIGS. 1 through 3. In addition, various stitching methods other than that shown in FIG. 9 can be utilized to secure the intermediate member 100 to the soft tissue 16. In any of these variations, the intermediate member 100 transfers load from near the end 120 of the soft tissue 16 to further away from the flexible anchor 12 and the intermediate member 100 reinforces the suture/tendon interfaces.

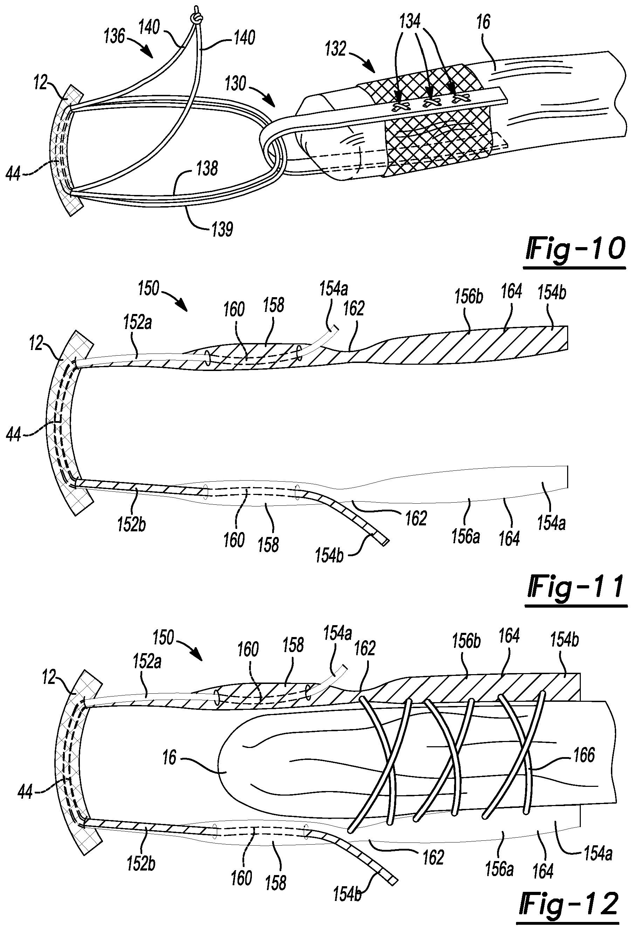

Referring to FIG. 10, an intermediate member 130 may be a thin, flat, planar ribbon or braid having a width that is greater than its thickness. The intermediate member 130 may be similar to the intermediate member 10 of FIG. 1. However, the intermediate member 10 of FIG. 1 may be sized to cover a larger surface area on the soft tissue 16, such as between 50 and 60 percent of the soft tissue surface area, to better distribute the load to the stitches 14. In contrast, the intermediate member 130 may be sized to cover a smaller surface area on the soft tissue 16, such as about 10 percent of the soft tissue surface area, to maximize the direct contact between the soft tissue 16 and bone and thereby promote integration.

In various implementations, an orthopedic matrix or mesh 132 may be wrapped around the soft tissue 16 and the intermediate member 130 may be placed over the mesh 132 on opposite sides of the soft tissue 16. Stitches 134 may be inserted through the intermediate member 130, through the mesh 132, and into the soft tissue 16 to secure the intermediate member 130 to the soft tissue 16. The stitching patterns disclosed above can be incorporated in any of the embodiments disclosed herein. Thus, for example, the stitches 14 shown in FIGS. 1 through 3 and/or the stitches 134 shown in FIG. 10 can incorporate one or more of the stitching patterns described with reference to FIGS. 4 through 6.

The mesh 132 may be a SportMesh™ Soft Tissue Reinforcement, available from Arthrotek®, a Biomet® company of Warsaw, Indiana. The mesh 132 may improve the distribution of load on the stitches 134 without increasing the amount of surface area on the soft tissue 16 that is completely covered. Further examples of orthopedic meshes are disclosed in U.S. Patent Application No. 2009/0318961 (see, e.g., FIGS. 36A, 36B, and 36C), filed on June 22, 2009.

The intermediate member 130 is shown connected to the flexible anchor 12 using a preformed adjustable self-locking suture construct 136 that includes a double-loop configuration with adjustable loops 138, 139 and ends 140. The adjustable loop 138 may be formed in the manner described above with respect to the suture construct 24. When the flexible anchor 12 is secured to bone, the ends 140 may be pulled to decrease the size of the adjustable loop 112 and thereby bring the intermediate member 100 closer to the flexible anchor 12. The intermediate member 130 may then be inserted partially through the adjustable loop 138 and secured to the soft tissue 16, and the ends 140 may be pulled to bring the soft tissue 16 closer to the flexible anchor 12.

Referring to FIGS. 11 and 12, a two-piece suture construct 150 is shown connected to the flexible anchor 12. The suture construct 150 includes a pair of separate sutures 152a, 152b having ends 154a, 154b and braided bodies 156a, 156b that form an intermediate member. Each of the braided bodies 156a, 156b includes a first portion 158 that defines a passage 160, a second portion 162, and a third portion 164. The first portion 158 may be a generally cylindrical braid, the third portion 164 may be a relatively flat braid such as a braided ribbon, and the second portion 162 may be a transition braid from the cylindrical braid to a flat braid. The first portion 158 may be wider than the second portion 162 and narrower than the third portion 164.

To form the suture construct 150, the suture 152a may be passed through the passage 160 in the braided body 156b, and the suture 152b may be passed through the passage 160 in the braided body 156a. The suture construct 150 may then be passed through the passage 44 in the flexible anchor 12 to position the braided bodies 156a, 156b on opposite sides of the passage 44 as shown. A suture 166 may then be stitched through the braided bodies 156a, 156b and the soft tissue 16 as shown in FIG. 12 to secure the suture construct 150 to the soft tissue 16. The suture 166 may be stitched using the crisscross pattern shown in FIG. 12 or other stitching techniques. Further examples of two-piece suture constructs are disclosed in U.S. Patent Application Publication No. 2012/0053630 (see, e.g., FIG. 2), filed on November 3, 2011.

Referring to FIGS. 13A and 13B, the flexible anchor 12, the suture construct 150, and the soft tissue 16 may then be inserted into a hole 168 drilled through cancellous bone 170 and cortical bone 172. The hole 168 may be a through bore that extends completely through the cancellous bone 170 and the cortical bone 172 as shown. Alternatively, the hole 168 may be a blind bore that extends only partially through the cancellous bone 170 and/or the cortical bone 172.

If the hole 168 is a through bore, the flexible anchor 12 may be pushed all of the way through the hole 168 and tension may be applied to the ends 154 of the suture 152 to set the flexible anchor 12 against the cortical bone 172. If the hole 168 is a blind bore, the flexible anchor 12 may be pushed into the hole 168 and tension may be applied to the ends 154 of the suture 152 to set the flexible anchor 12 against the cancellous bone 170 and/or the cortical bone 172. Once the flexible anchor 12 is set, applying tension to the ends 154 pulls the soft tissue 16 further into the hole 168. Although FIGS. 13A and 13B illustrating attaching soft tissue to bone using the suture construct 150, other embodiments of suture constructs and intermediate members disclosed herein may be used in a similar manner to attach soft tissue to bone.

The foregoing description of the embodiments has been provided for purposes of illustration and description. It is not intended to be exhaustive or to limit the disclosure. Individual elements or features of a particular embodiment are generally not limited to that particular embodiment, but, where applicable, are interchangeable and can be used in a selected embodiment, even if not specifically shown or described. The same may also be varied in many ways. Such variations are not to be regarded as a departure from the disclosure, and all such modifications are intended to be included within the scope of the disclosure.

## Claims

1. An apparatus for repairing a tear in soft tissue (16) comprising:
a bone anchor (12) defining a passage (22, 44) that extends through the bone anchor (12);
a suture construct (24, 58, 110) including a first suture (32) that extends through the passage (22, 44) in the bone anchor (12) to form an adjustable loop (28, 112, 113);
an intermediate member (10, 80, 100, 130) configured to be inserted partially through the adjustable loop (28, 30) and folded to form first and second segments (18, 20, 80a, 80b) that are placeable on the soft tissue (16); and
a second suture (14, 82, 134, 116, 118) that is configured to be passed through the first and second segments (18, 20, 80a, 80b) of the intermediate member (10, 80, 100, 130) and the soft tissue (16) to form stiches that secure the intermediate member (10, 80, 100, 130) to the soft tissue (16), wherein the intermediate member (10, 80, 100, 130) is configured to increase pull-out forces of the stitches and transfer load from those of the stitches disposed closest to the bone anchor (12) to those of the stitches disposed further away from the bone anchor (12).

2. The apparatus of claim 1 wherein the intermediate member (10, 80, 100, 130) defines preformed openings (102) for receiving the second suture (14) as the second suture (14) is passed in and out of the soft tissue (16) to stitch the intermediate member (10, 80, 100, 130) to the soft tissue (16).

3. The apparatus of claim 1 further comprising needles (108) and third and fourth sutures, the third and fourth sutures (104) extending from opposite ends of the intermediate member (10, 80, 100, 130) and connecting the needles (108) to the intermediate member (10, 80, 100, 130).

4. The apparatus of claim 1 wherein the intermediate member (10, 80, 100, 130) is a flat, planar member that includes at least one of a ribbon and a sheath.

5. The apparatus of claim 1 wherein the intermediate member (10, 80, 100, 130) includes at least one of polyester, polyethylene, polyurethane urea, polyether ether ketone (PEEK), and bioresorbable material.

6. The apparatus of claim 1 wherein the intermediate member (10, 80, 100, 130) is at least one of braided, knit, and woven.

7. The apparatus of claim 1 wherein the second suture (14) includes a plurality of suture constructs (58, 69) configured to pass through the intermediate member (10, 80, 100, 130) and the soft tissue (16) such that a pair of anchors included in each of the suture constructs (58, 69) are configured to be disposed on opposite sides of the soft tissue (16) to form an inline attachment configuration.

8. The apparatus of claim 1 wherein the second suture (14) includes a plurality of suture constructs (58, 69) configured to pass through the intermediate member (10, 80, 100, 130) and the soft tissue (16) and such that a pair of anchors (60, 62) included in each of the suture constructs (58, 69) are configured to be disposed on one side of the soft tissue (16) to form a u-shaped configuration.

9. The apparatus of claim 1 wherein the second suture (14) includes:
a plurality of suture constructs (58, 69) configured to pass through the intermediate member (10, 80, 100, 130) and the soft tissue (16) such that adjustable loops (66, 67) included in each of the suture constructs (58, 69) are configured to be disposed on opposite sides of the soft tissue (16); and
locking members (90) configured to be inserted into the adjustable loops (66, 67) to prevent the adjustable loops (66, 67) from being pulled through the intermediate member (10, 80, 100, 130) as the adjustable loops (66, 67) are tightened.

10. The apparatus of claim 1 wherein the bone anchor (12) is flexible and pulling the ends of the second suture (14) sets the flexible bone anchor (12) against the bone.

11. An apparatus for repairing a tear in soft tissue (16) comprising:
a bone anchor (12) defining a first passage (44); and
a suture construct (150) including a first suture (152a) having a first braided body (156a) and a second suture (152b) having a first braided body (156b), the second suture (152b) extending through a second passage (160) in the first braided body (156a), the first suture (152a) extending through a third passage (160) in the first braided body (156b), the first and second sutures (152a, 152b) extending through the first passage (44) in the bone anchor (12) such that the first and second braided bodies (156a, 156b) are disposed on opposite sides of the first passage (44), the first and second braided bodies (156a, 156b) each including a first portion (158) that defines one of the second and third passages (160) and a second portion (162) that extends from the first portion (158) in a direction away from the bone anchor (12), the first portion (158) having a cylindrical shape and the second portion having a flat shape.

12. The apparatus of claim 11 wherein the bone anchor (12) is a flexible anchor.

13. The apparatus of claim 11, further comprising a second suture (14) that is configured to be passed through the second portion of each of the first and second braided bodies and soft tissue (16) to secure the suture construct (24) to the soft tissue (16).

14. The apparatus of claim 11, wherein the bone anchor (12) is a flexible anchor and the suture construct (24) passes through a first opening in a sidewall of the bone anchor (12) along the first passage (22, 44) and out a second opening in the sidewall of the anchor.

## Patentansprüche

1. Vorrichtung zur Instandsetzung eines Risses in weichem Gewebe (16), umfassend:
einen Knochenanker (12), der einen Durchgang (22, 44) definiert, der sich durch den Knochenanker (12) erstreckt;
eine Nahtkonstruktion (24, 58, 110) mit einer ersten Naht (32), die sich durch den Durchgang (22, 44) in dem Knochenanker (12) erstreckt, um eine verstellbare Schlaufe (28, 112, 113) zu bilden;
ein intermediäres Element (10, 80, 100, 130), das so gestaltet ist, dass es teilweise durch die verstellbare Schlaufe (28, 30) eingeführt und gefaltet werden kann, um erste und zweite Segmente (18, 20, 80a, 80b) zu bilden, die an dem weichen Gewebe (16) platziert werden können; und
eine zweite Naht (14, 82, 134, 116, 118), die so gestaltet ist, dass sie durch die ersten und zweiten Segmente (18, 20, 80a, 80b) des intermediären Elements (10, 80, 100. 130) und das weiche Gewebe (16) geführt werden kann, um Stiche zu bilden, die das intermediäre Element (10, 80, 100, 130) an dem weichen Gewebe (16) sichern, wobei das intermediäre Element (10, 80, 100, 130) so gestaltet ist, dass es die Ausziehkräfte der Stiche erhöht und die Belastung von den Stichen, die am nächsten an dem Knochenanker (12) angeordnet sind, auf die Stiche überträgt, die am weitesten von dem Knochenanker (12) entfernt angeordnet sind.

2. Vorrichtung nach Anspruch 1, wobei das intermediäre Element (10, 80, 100, 130) vorgeformte Öffnungen (102) zur Aufnahme der zweiten Naht (14) definiert, wenn die zweite Naht (14) in das weiche Gewebe (16) und aus diesem heraus geführt wird, um das intermediäre Element (10, 80, 100, 130) an das weiche Gewebe (16) zu nähen.

3. Vorrichtung nach Anspruch 1, ferner umfassend Nadeln (108) und eine dritte und eine vierte Naht, wobei sich die dritte und die vierte Naht (104) von entgegengesetzten Enden des intermediären Elements (10, 80, 100, 130) erstrecken und die Nadeln (108) mit dem intermediären Element (10, 80, 100, 130) verbinden.

4. Vorrichtung nach Anspruch 1, wobei das intermediäre Element (10, 80, 100, 130) ein flaches, planares Element ist, das wenigstens ein Band oder eine Hülse aufweist.

5. Vorrichtung nach Anspruch 1, wobei das intermediäre Element (10, 80, 100, 130) wenigstens eines der folgenden aufweist: Polyester, Polyethylen, Polyurethanharnstoff, Polyetheretherketon (PEEK) und bioresorbierbares Material.

6. Vorrichtung nach Anspruch 1, wobei das intermediäre Element (10, 80, 100, 130) wenigstens geflochten, gestrickt oder gewebt ist.

7. Vorrichtung nach Anspruch 1, wobei die zweite Naht (14) eine Mehrzahl von Nahtkonstruktionen (58, 69) aufweist, die so gestaltet sind, dass sie durch das intermediäre Element (10, 80, 100, 130) und das weiche Gewebe (16) verlaufen, so dass ein Ankerpaar , das in jeder der Nahtkonstruktionen (58, 59) enthalten ist, so gestaltet ist, dass es auf entgegengesetzten Seiten des weichen Gewebes (16) angeordnet werden kann, um eine lineare Befestigungskonfiguration zu bilden.

8. Vorrichtung nach Anspruch 1, wobei die zweite Naht (14) eine Mehrzahl von Nahtkonstruktionen (58, 69) aufweist, die so gestaltet sind, dass sie durch das intermediäre Element (10, 80, 100, 130) und das weiche Gewebe (16) verlaufen und dass ein Ankerpaar (60, 62), das in jeder der Nahtkonstruktionen (58, 69) enthalten ist, so gestaltet ist, dass es auf einer Seite des weichen Gewebes (16) angeordnet werden kann, um eine U-förmige Konfiguration zu bilden.

9. Vorrichtung nach Anspruch 1, wobei die zweite Naht (14) folgendes aufweist:
eine Mehrzahl von Nahtkonstruktionen (58, 69), die so gestaltet sind, dass sie durch das intermediäre Element (10, 80, 100, 130) und das weiche Gewebe (16) verlaufen, so dass die verstellbaren Schlaufen (66, 67), die jede der Nahtkonstruktionen (58, 69) aufweist, so gestaltet sind, dass sie auf entgegengesetzten Seiten des weichen Gewebes (16) angeordnet werden können; und
Verriegelungselemente (90), die so gestaltet sind, dass sie in die verstellbaren Schlaufen (66, 67) eingeführt werden können, um zu verhindern, dass die verstellbaren Schlaufen (66, 67) durch das intermediäre Element (10, 80, 100, 130) gezogen werden, wenn die verstellbaren Schlaufen (66, 67) festgezogen werden.

10. Vorrichtung nach Anspruch 1, wobei der Knochenanker (12) flexibel ist, und wobei der flexible Knochenanker (12) durch Ziehen der Enden der zweiten Naht (14) an dem Knochen festgesetzt wird.

11. Vorrichtung zur Instandsetzung eines Risses in weichem Gewebe (16), umfassend:
einen Knochenanker (12), der einen ersten Durchgang (44) definiert; und
eine Nahtkonstruktion (150) mit einer ersten Naht (152a) mit einem ersten geflochtenen Körper (156a) und mit einer zweiten Naht (152b) mit einem ersten geflochtenen Körper (156b), wobei sich die zweite Naht (152b) durch einen zweiten Durchgang (160) in dem ersten geflochtenen Körper (156a) erstreckt, wobei sich die erste Naht (152a) durch einen dritten Durchgang (160) in dem ersten geflochtenen Körper (156b) erstreckt, wobei sich die erste und die zweite Naht (152a, 152b) durch den ersten Durchgang (44) in dem Knochenanker (12) erstrecken, so dass die ersten und zweiten geflochtenen Körper (156a, 156b) auf entgegengesetzten Seiten des ersten Durchgangs (44) angeordnet sind, wobei die ersten und zweiten geflochtenen Körper (156a, 156b) jeweils einen ersten Teil (158) aufweisen, der einen der zweiten und dritten Durchgänge (16) definiert, und einen zweiten Teil (162), der sich von dem ersten Teil (158) in eine von dem Knochenanker (12) weggehende Richtung erstreckt, wobei der erste Teil (158) eine zylindrische Form aufweist, und wobei der zweite Teil eine flache Form aufweist.

12. Vorrichtung nach Anspruch 11, wobei der Knochenanker (12) ein flexibler Anker ist.

13. Vorrichtung nach Anspruch 11, wobei diese ferner eine zweite Naht (14) umfasst, die so gestaltet ist, dass sie durch den zweiten Teil jedes der ersten und zweiten geflochtenen Körper und das weiche Gewebe (15) geführt werden kann, um die Nahtkonstruktion (24) an dem weichen Gewebe (16) zu sichern.

14. Vorrichtung nach Anspruch 11, wobei der Knochenanker (12) ein flexibler Anker ist, und wobei die Nahtkonstruktion (24) durch eine erste Öffnung in einer Seitenwand des Knochenankers (12) entlang dem ersten Durchgang (22, 44) und aus einer zweiten Öffnung in der Seitenwand des Ankers verläuft.

## Revendications

1. Appareil pour réparer une déchirure dans un tissu mou (16) comprenant :
un ancrage osseux (12) définissant un passage (22, 44) qui s'étend à travers l'ancrage osseux (12) ;
une construction de suture (24, 58, 110) comprenant une première suture (32) qui s'étend à travers le passage (22, 44) dans l'ancrage osseux (12) pour former une boucle réglable (28, 112, 113) ;
un composant intermédiaire (10, 80, 100, 130) conçu pour être inséré partiellement à travers la boucle réglable (28, 30) et plié pour former des premier et second segments (18, 20, 80a, 80b) qui peuvent être placés sur le tissu mou (16) ; et
une deuxième suture (14, 82, 134, 116, 118) qui est conçue pour être passée à travers les premier et second segments (18, 20, 80a, 80b) du composant intermédiaire (10, 80, 100, 130) et le tissu mou (16) pour former des points de suture qui fixent le composant intermédiaire (10, 80, 100, 130) au tissu mou (16), le composant intermédiaire (10, 80, 100, 130) étant conçu pour accroître les forces d'arrachement des points de suture et la charge de transfert des points de suture situés le plus près de l'ancrage osseux (12) aux points de suture situés le plus loin de l'ancrage osseux (12).

2. Appareil selon la revendication 1, le composant intermédiaire (10, 80, 100, 130) définissant des ouvertures préformées (102) pour recevoir la deuxième suture (14) alors que la deuxième suture (14) est entrée et sortie du tissu mou (16) pour suturer le composant intermédiaire (10, 80, 100, 130) sur le tissu mou (16).

3. Appareil selon la revendication 1 comprenant en outre des aiguilles (108) et des troisième et quatrième sutures, les troisième et quatrième sutures (104) s'étendant à partir d'extrémité opposée du composant intermédiaire (10, 80, 100, 130) et reliant les aiguilles (108) au composant intermédiaire (10, 80, 100, 130).

4. Appareil selon la revendication 1, le composant intermédiaire (10, 80, 100, 130) étant un élément plan plat qui comprend au moins un ruban ou une gaine.

5. Appareil selon la revendication 1, le composant intermédiaire (10, 80, 100, 130) comprenant du polyester, du polyéthylène, du polyuréthanne-urée, du polyéther-éther-cétone (PEEK) et/ou un matériau biorésorbable.

6. Appareil selon la revendication 1, le composant intermédiaire (10, 80, 100, 130) étant tressé, tricoté et/ou tissé.

7. Appareil selon la revendication 1, la deuxième suture (14) comprenant une pluralité de constructions de suture (58, 69) conçues pour passer à travers le composant intermédiaire (10, 80, 100, 130) et le tissu mou (16) de sorte qu'une paire d'ancrages inclus dans chacune des constructions de suture (58, 69) soit conçue pour être disposée sur des côtés opposés du tissu mou (16) pour former une configuration de fixation en ligne.

8. Appareil selon la revendication 1, la deuxième suture (14) comprenant une pluralité de constructions de suture (58, 69) conçues pour passer à travers le composant intermédiaire (10, 80, 100, 130) et le tissu mou (16) et de sorte qu'une paire d'ancrages (60, 62) incluse dans chacune des constructions de suture (58, 69) soit conçue pour être disposée sur un côté du tissu mou (16) pour former une configuration en forme de U.

9. Appareil selon la revendication 1, la deuxième suture (14) comprenant :
une pluralité de constructions de suture (58, 69) conçues pour passer à travers le composant intermédiaire (10, 80, 100, 130) et le tissu mou (16) de sorte que des boucles réglables (66, 67) incluses dans chacune des constructions de suture (58, 69) soient conçues pour être disposées sur des côtés opposés du tissu mou (16) ; et
des éléments de verrouillage (90) conçus pour être insérés dans les boucles réglables (66, 67) pour éviter que les boucles réglables (66, 67) ne soient tirées à travers le composant intermédiaire (10, 80, 100, 130) alors que les boucles réglables (66, 67) sont serrées.

10. Appareil selon la revendication 1, l'ancrage osseux (12) étant flexible et en tirant les extrémités de la deuxième suture (14) place l'ancrage osseux (12) flexible contre l'os.

11. Appareil pour réparer une déchirure dans un tissu mou (16) comprenant :
un ancrage osseux (12) définissant un premier passage (44) ; et
une construction de suture (150) comprenant une première suture (152a) ayant un premier corps tressé (156a) et une deuxième suture (152b) ayant un premier corps tressé (156b), la deuxième suture (152b) s'étendant à travers un deuxième passage (160) dans le premier corps tressé (156a), la première suture (152a) s'étendant à travers un troisième passage (160) dans le premier corps tressé (156b), les première et deuxième sutures (152a, 152b) s'étendant à travers le premier passage (44) dans l'ancrage osseux (12) de sorte que les premier et second corps tressés (156a, 156b) soient disposés sur des côtés opposés du premier passage (44), les premier et second corps tressés (156a, 156b) comprenant chacun une première partie (158) qui définit l'un des deuxième et troisième passages (160) et une seconde partie (162), qui s'étend à partir de la première partie (158) dans une direction opposée à l'ancrage osseux (12), la première partie (158) ayant une forme cylindrique et la seconde partie ayant une forme plate.

12. Appareil selon la revendication 11, l'ancrage osseux (12) étant un ancrage flexible.

13. Appareil selon la revendication 11, comprenant en outre une deuxième suture (14) qui est conçue pour être passée à travers la seconde partie de chacun des premier et deuxième corps tressés et le tissu mou (16) pour fixer la construction de suture (24) au tissu mou (16).

14. Appareil selon la revendication 11, l'ancrage osseux (12) étant un ancrage flexible et la construction de suture (24) passant à travers une première ouverture dans une paroi latérale de l'ancrage osseux (12) le long du premier passage (22, 44) et hors d'une seconde ouverture dans la paroi latérale de l'ancrage.
